# EUROPÄISCHE PATENTANMELDUNG

(11) **EP 0 547 402 A2**
(43) Veröffentlichungstag der Anmeldung: **23.06.1993**
(21) Anmeldenummer: 92119879.2
(22) Anmeldetag: 23.11.1992
(51) Int. Cl.: C07C 213/04

(54) **Verfahren zur Herstellung von lagerstabilen 1-Di(C1-bis C3-)alkylamino-2,3-propandiolen**

(30) Priorität: 14.12.1991 DE 4141349
(71) Anmelder: Degussa Aktiengesellschaft, D-60311 Frankfurt (DE)
(72) Erfinder: Götz, Peter, Dr., W6365 Rosbach (DE); Möhlenkamp, Alois, W-6458 Rodenbach (DE)

(57) **Zusammenfassung**

Aus Glycid und Di(C₁- bis C₃-)alkylaminen lassen sich in bekannter Weise 1-Di(C₁- bis C₃-)alkylamino-2,3-propandiole herstellen. Die hieraus resultierenden, an sich farblosen Verbindungen verfärben sich bei der Lagerung. Dieser Nachteil läßt sich erfindungsgemäß durch eine 0,5- bis 5-stündige thermische Behandlung bei 150 bis 200 ^{o}C vor der Reindestillation des Dialkylaminopropandiols beheben. Das Verfahren richtet sich insbesondere auf die Herstellung von lagerstabilem 1-Dimethylamino-2,3-propandiol.

## Beschreibung

Die Erfindung betrifft ein Verfahren zur Herstellung von lagerstabilen 1-Di(C₁ - bis C₃-)alkylamino-2,3-propandiolen, insbesondere Dimethylaminopropandiol, aus Glycid und den entsprechenden Di(C₁- bis C₃)alkylaminen. Erfindungsgemäß hergestellte Produkte verfärben sich bei der Lagerung praktisch nicht.

1-Dialkylamino-2,3-propandiole lassen sich in bekannter Weise aus Glycerinmonochlorhydrin und einem Dialkylamin herstellen. Nach diesem Verfahren hergestellte 1-Di(C₁- bis C₃)alkylamino-2,3-propandiole sind zwar lagerstabil, daß heißt, sie verfärben sich bei der Lagerung kaum, jedoch ist das Verfahren durch den Anfall eines Salzes aus der abgespaltenen Chlorwasserstoffsäure in ökologischer Hinsicht belastet. Gemäß einem Alternativverfahren, wobei als Reaktionspartner anstelle des Glycerinmonochlorhydrins Glycid (2,3-Epoxypropanol) eingesetzt wird, lassen sich die entsprechenden Dialkylaminopropandiole in hoher Ausbeute ohne jeglichen Salzanfall herstellen. Die Herstellung von Dialkylaminopropandiolen aus Glycid und sekundären Aminen ist ausführlich beschrieben worden - siehe Glycidol, Properties reactions and applications von A. Kleemann und R. Wagner, A. Hüthig Verlag (1981), Seiten 90-94. Die Reaktionspartner können äquimolar eingesetzt werden; wie im Falle primärer Amine oder Ammoniak ist aber ein Aminüberschuß vorteilhaft. Die Umsetzung kann in Gegenwart von inerten Lösungsmitteln oder im Falle eines Aminüberschusses in deren Abwesenheit durchgeführt werden (vgl. EP-B 0 038 409, DE 30 14 098 C2).

Ein bisher nicht erklärbarer Nachteil des Verfahrens via Glycid besteht darin, daß sich so hergestellte niedere Dialkylaminopropandiole, inbesondere Dimethylaminopropandiol, bei der Lagerung gelb verfärben. Diese Verfärbung wird bereits kurze Zeit nach der Destillation beobachtet. Die Verfärbung ist zunächst blaßgelb, intensiviert sich dann innerhalb weniger Tage bis zu einem Maximum und läßt sich weder durch erhöhten Destillationsaufwand noch durch Maßnahmen wie Luft- und Lichtausschluß unterdrücken.

Aufgabe der vorliegenden Erfindung ist demgemäß, das an sich bekannte Verfahren zur Herstellung von 1-Di(C₁- bis C₃-)alkylamino-2,3-propandiolen aus Glycid und entsprechenden Di-(C₁- bis C₃-)alkylamin durch Umsetzung des Glycid mit dem Di-(C₁- bis C₃)-alkylamin im Molverhältnis von 1 zu größer 1 in An- oder Abwesenheit eines Lösungsmittels und destillative Aufarbeitung des Destillationsgemischs derart zu verbessern, daß man lagerstabile, das heißt, praktisch nicht verfärbene 1-Di(C₁- bis C₃)-alkylamino-2,3-propandiole erhält. Es wurde gefunden, daß sich diese Aufgabe dadurch lösen läßt, daß man das von nicht umgesetztem Amin und, soweit vorhanden, Lösungsmitteln im wesentlichen befreite Reaktionsgemisch 0,5 bis 5 Stunden bei 50 bis 200 °C thermisch behandelt und anschließend destilliert.

Die thermische Behandlung ist von besonderer Bedeutung zur Herstellung von 1-Dimethylamino-2,3-propandiol, das als Ausgangsprodukt zur Herstellung von Textilhilfsmitteln Bedeutung erlangt hat. Vorzugsweise erfolgt die thermische Behandlung im Falle des 1-Dimethylamino-2,3-propandiols bei 160 bis 190 ^{o}C innerhalb 1 bis 3 Stunden.

Die Umsetzung zwischen Glycid und dem Dialkylamin erfolgt bei einem Molverhältnis von 1 zu größer 1 zweckmäßigerweise 1 zu größer 2, insbesondere 1 zu 2 bis 3. Die eingesetzten Dialkylamine enthalten 2 bis 6 C-Atome, Dimethylamin und Diethylamin werden bevorzugt, einsetzbar sind aber auch Dialkylamine mit unterschiedlichen Alkylgruppen. Der Aminüberschuß dient gleichzeitig als Lösungsmittel. Zudem können bei der Umsetzung aber auch andere Lösungsmittel zugegen sein, wie beispielsweise organische Lösungsmittel aus der Reihe niedrig siedender Alkohole und Ether oder Wasser. Vorzugsweise werden außer dem Aminüberschuß und gegebenenfalls Wasser keine weiteren organischen Lösungsmittel eingesetzt. Ein im wesentlichen wasserfreies Reaktionsmedium wird im Hinblick auf die vereinfachte Aufarbeitung bevorzugt. Die Reaktion kann in einer kontinuierlichen Apparatur oder auch diskontinuierlich durchgeführt werden.

Um eine Polymerisation des Glycids zu vermeiden, wird stets Glycid zum Amin dosiert und nicht umgekehrt. Die Reaktionstemperatur wird zweckmäßigerweise unter 100 ^{o}C, insbesondere im Bereich von 30 bis 60 ^{o}C, liegen. Bei Verwendung von Dialkylaminen mit einem Siedepunkt unter 60 ^{o}C wird die Umsetzung zweckmäßigerweise bei dem sich bei der gewählten Reaktionstemperatur einstellenden Druck durchgeführt.

Nach beendeter Umsetzung wird das Reaktionsgemisch von nicht umgesetztem Amin und, soweit vorhanden, Lösungsmitteln weitgehend befreit; dies erfolgt mittels bekannter destillativer Maßnahmen und/oder durch desorptive Maßnahmen unter Verwendung von Inertgasen. Die thermische Behandlung des von niedrig siedenden Bestandteilen im wesentlichen befreiten Reaktionsgemisches erfolgt bei Normaldruck oder erhöhtem Druck.

Erfindungsgemäß thermisch behandeltes 1-Di(C₁- bis C₃-)alkylamino-2,3-propandiol ist nach der Destillation praktisch farblos und ändert seine Farbe auch nach längerer Lagerung nicht. Es war nicht vorhersehbar, daß durch eine einfache thermische Behandlung des Roh-Dialkylaminopropandiols bzw. des Reaktionsgemischs aus seiner Herstellung via Glycid die im vorbekannten Verfahren als sehr nachteilig erkannte Verfärbung bei der Lagerung vermieden werden konnte.

Überraschenderweise führt die thermische Behandlung zu keinem feststellbaren Ausbeuteverlust.

### Beispiel 1

### Herstellung von 1-Dimethylamino-2,3-propandiol mit Wasser als Lösungsmittel

### Einsatzstoffe:

683 g = 5 Mol Dimethylamin als 33%ige Lösung in Wasser
296 g = 4 Mol Glycid

### Durchführung der Reaktion:

Das wäßrige Amin wird in einem Kolben vorgelegt und auf 50 ^{o}C erwärmt. Dann wird unter Rühren das Glycid innerhalb von 3 h so zudosiert, daß die Temperatur nicht über 75 ^{o}C steigt. Anschließend wird weitere 2 h bei 50 ^{o}C gerührt. Aus dem Reaktionsgemiseh wird zunächst das Wasser und überschüssiges Amin destillativ entfernt. Das Rohprodukt wird dann bei Normaldruck für 2 h auf 175 ^{o}C erwärmt, anschließend wieder auf 50 ^{o}C abgekühlt. Danach wird das 1-Dimethylaminopropandiol bei 10 mbar und einer Kopftemperatur von 90 ^{o}C destilliert. Man erhält 343 g Dimethylaminopropandiol, was 72 % d. Th. Ausbeute, bezogen auf eingesetztes Glycid, entspricht.

### Farbzahlen:

| | |
|---|---|
| unmittelbar nach der Destillation | 50 Hazen |
| nach 6 Monaten Lagerung (ohne besondere Vorkehrungen) | 80 Hazen |

### Beispiel 2

### Herstellung von 1-Dimethylamino-2,3-propandiol ohne Lösungmsittel

### Einsatzstoffe:

77,6 kg = 114 l = 1,72 KMol Dimethylamin (wasserfrei)
51,0 kg = 45,8 l = 0,69 KMol Glycid

### Durchführung der Reaktion:

Das Dimethylamin wird in einem mit Rührer und Dosierpumpe ausgestatteten Druckreaktor (160 l Nutzvolumen) vorgelegt und auf 40 ^{o}C erwärmt, wobei sich ein Druck von 3 bis 4 bar einstellt. Innerhalb von 4,5 h wird das Glycid so zudosiert, daß eine Temperatur von 40 ^{o}C nicht überschritten wird. Der Druck fällt dabei ab. Nach der Zudosierung wird 1 h bei 40 ^{o}C gerührt, um das Glycid vollständig umzusetzen. Danach wird die Temperatur auf 60 ^{o}C erhöht und der größte Teil des überschüssigen Amins (ca. 42 kg) entfernt. Zur Abtrennung des restlichen Dimethylamins wird das Reaktionsgemisch im Destillationsgefäß bei 60 ^{o}C Sumpftemperatur und 10 mbar Druck behandelt. Das Rohproduckt wird bei Normaldruck für 2 Stunden auf 175 ^{o}C erwärmt, anschließend wieder auf 60 ^{o}C abgekühlt. Anschließend wird das Dimethylaminopropandiol bei 10 mbar und einer Kopftemperatur von 90 ^{o}C destilliert. Man erhält 75,2 kg Dimethylaminopropandiol, was 92 % d. Th., Ausbeute bezogen auf eingesetztes Glycid, entspricht.

### Farbzahlen:

| | |
|---|---|
| unmittelbar nach der Destillation | 30 Hazen |
| nach 6 Monaten Lagerung (ohne besondere Vorkehrungen) | 80 Hazen |
| nach 6 Monaten Lagerung unter Luft- und Lichtausschluß | 45 Hazen |

### Vergleichsbeispiel

1-Dimethylamino-2,3-propandiol wurde analog Beispiel 2 hergestellt, wobei jedoch die thermische Behandlung bei 175 ^{o}C nicht durchgeführt wurde. Die Ausbeute beträgt 92 % d. Th.

### Farbzahlen:

| | |
|---|---|
| unmittelbar nach der Destillation | 150 Hazen |
| nach 6 Monaten Lagerung (ohne besondere Vorkehrungen) | >1000 Hazen |
| nach 6 Monaten Lagerung unter Luft- und Lichtausschluß | 400 Hazen |

## Patentansprüche

1. Verfahren zur Herstellung von lagerstabilen 1-Di(C₁- bis C₃-)alkylamino-2,3-propandiolen durch Umsetzung von Glycid mit symmetrischen oder unsymmetrischen Di(C₁- bis C₃-)alkylaminen im Molverhältnis von 1 zu größer 1 in An- oder Abwesenheit eines Lösungsmittels und destillative Aufarbeitung des Reaktionsgemischs,
dadurch gekennzeichnet,
daß man das von nicht umgesetztem Amin und, soweit vorhanden, Lösungsmitteln im wesentlichen befreite Reaktionsgemisch 0,5 bis 5 Stunden bei 150 ^{o}C bis 200 ^{o}C thermisch behandelt und anschließend destilliert.

2. Verfahren nach Anspruch 1,
dadurch gekennzeichnet,
daß man 1-Dimethylamino-2,3-propandiol aus Glycid und Dimethylamin herstellt, wobei die Umsetzung Vorzugsweise in Abwesenheit eines anderen Lösungsmittels als des Dimethylamins und gegebenenfalls Wasser durchgeführt wird.

3. Verfahren nach Anspruch 2,
dadurch gekennzeichnet,
daß man die thermische Behandlung 1 bis 2 Stunden bei 160 ^{o}C bis 190 ^{o}C durchführt.
